# EUROPEAN PATENT APPLICATION

(11) **EP 3 279 667 A1**
(43) Date of publication of application: **07.02.2018**
(21) Application number: 16306004.9
(22) Date of filing: 02.08.2016
(51) Int. Cl.: G01N 33/68

(54) **CARDIAC TROPONIN I AND COPEPTIN AS BIOMARKERS FOR SHORT-TERM MORTALITY IN ACUTE EXACERBATION OF CHRONIC OBSTRUCTIVE PULMONARY DISEASE (AECOPD)**

(71) Applicant: ABBOTT LABORATORIES, Abbott Park, IL 60064-3500 (US); INSTITUT NATIONAL DE LA SANTE ET DE LA RECHERCHE MEDICALE (INSERM), 75654 Paris Cédex (FR); Assistance Publique - Hôpitaux De Paris, 75475 Paris Cedex 10 (FR); Université Paris Diderot - Paris 7, 75013 Paris (FR)
(72) Inventor: BESHIRI, Agim, Abbott Park, IL 60064 (US); MEBAZAA, Alexandre, 92120 Montrouge (FR); LARIBI, Said, 75654 Paris (FR)
(74) Representative: Marro, Nicolas

(57) **Abstract**

The invention provides methods of detecting cardiac troponin (cTn) and copeptin proteins in samples from patients suffering from an acute exacerbation of COPD (AECOPD) in order to determine risk of short-term mortality.

## Description

### TECHNICAL FIELD

This disclosure relates to methods for determining risk of short-term mortality in acute exacerbated chronic obstructive pulmonary disease (AECOPD).

### BACKGROUND OF THE INVENTION

Chronic Obstructive Pulmonary Disease (COPD) is projected to be the third leading cause of death worldwide by 2030 due to smoking and the aging population (Pauwels et al., Respir Care, 46(8): 798-825 (2001); and Mathers et al., PLoS Med; 3(11): e442 (2006)). Acute exacerbations of COPD (AECOPD) describe the phenomenon of sudden worsening in airway function and respiratory symptoms in patients with COPD and represent important episodes of disease progression (Rabe et al., Am. J. Respir. Crit. Care. Med, 176(6): 532-55 (2007)). Mortality rates of patients hospitalized for AECOPD (i.e., "in-hospital mortality") range from 2.5% up to 24.5% for severe cases requiring ICU care (see, e.g., Patil et al., Arch. Intern. Med.; 163(10): 1180-6 (2003); Jenkins et al., Respir. Res., 10: 59 (2009); Steer et al., Thorax, 67(2): 117-21 (2012); Perera et al., COPD, 9(2): 131-41 (2012); and Ai-Ping et al., Chest, 128(2): 518-24(2005)). High mortality in AECOPD patients has been associated with a number of factors, such as, for example, number of episodes of AECOPD, co-morbidities, age, severity of dyspnea, and body-mass-index (BMI) (see, e.g., Roche et al., Eur. Respir. J., 32(4): 953-61 (2008); Connors et al., Am J Respir Crit Care Med, 154(4 Pt 1): 959-67 (1996); and Shah et al., Eur Heart J, 33(17): 2197-205 (2012)). The CURB-65 score, which is a prognosticator used to stratify community acquired pneumonia patients presenting to the emergency room (see, e.g., Lim et al., Thorax, 58(5): 377-382 (2003) and Lim et al., Thorax, 64 Suppl 3: 1-55 (2009)), also has been associated with in-hospital and 30-day mortality after AECOPD in both retrospective and prospective studies (see, e.g., Chang et al., Internal Medicine Journal, 37(4): 236-241 (2007) and Chang et al., Respirology, 16(1): 146-151 (2011)).

The cause of death in AECOPD patients has been primarily attributed to an aggravation of lung function, which has led to recommendations for ventilator support (noninvasive or invasive), rapid-acting inhaled bronchodilators, systemic corticosteroids, and antibiotics in order to prevent in-hospital death (see, e.g., Ferguson, G.T. Chest, 117(2 Suppl): 23S-8S (2000)). However, when obstructive lung disease (i.e., COPD or asthma) is not the underlying cause of death, the most common cause of death listed on death certificates is ischemic heart disease (see, e.g., Hansell et al., Eur Respir J, 22(5): 809-14 (2003)).

COPD is increasingly described as a combined pulmonary and vascular disease. Indeed, major risk factors, including age and smoking, overlap for COPD and vascular diseases. Common genetic determinants for COPD and coronary artery disease in humans also have recently been identified (see, e.g., Sabater-Lleal et al., PLoS One, 9(8): e104082 (2014)). In addition, plasma biomarkers measured at hospital admission are increasingly used to evaluate disease severity. In AECOPD patients, an association between elevated cardiac troponin (measured by high-sensitivity assays) at hospital admission and death has been reported (see, e.g., Stolz et al., Chest, 131(4): 1058-67 (2007); Chang et al., Thorax, 66(9): 764-8 (2011); Rahim et al., Egyptian J. Chest Diseases and Tuberculosis, 62: 549-555 (2013); Soyseth et al., Heart, 99(2): 122-126 (2013); and Harvey, M.G. and Hancox, R.J., Emerg. Med. Australasia, 16(3): 212-215 (2004)).

While elevated cardiac troponin has been demonstrated to be a prognosticator of acute myocardial injury (AMI), both alone and in combination with other biomarkers (e.g., procalcitonin (PCT) and/or c-reactive protein (CRP), and copeptin) (see, e.g., Seronde et al., Int J Cardiol, 168(4): 3404-11 (2013); Schuetz et al., Chest, 141(4): 1063-73 (2012); and Wildi et al., Int J Cardiol, 190: 170-6 (2015)), the impact of elevated cardiac troponin, potentially combined with other biomarkers, on myocardial injury and short-term death in AECOPD patients remains unknown.

Thus, there is a need for methods determining risk of short-term mortality in patients suffering from AECOPD. The invention described herein provides such methods.

### BRIEF SUMMARY OF THE INVENTION

The invention provides a method of detecting cardiac troponin (cTn) and copeptin proteins in a sample obtained from a patient diagnosed with chronic obstructive pulmonary disease (COPD), which method comprises (a) obtaining a blood sample from a patient diagnosed with COPD, and (b) detecting whether cardiac troponin and copeptin are present in the blood sample by contacting the blood sample with an anti-cardiac troponin antibody and an anti-copeptin antibody.

In one aspect of the above method, the patient is diagnosed with an acute exacerbation of COPD (AECOPD).

The invention also provides a method of determining risk of short-term mortality in a patient having an acute exacerbation of COPD (AECOPD), which method comprises (a) obtaining a blood sample from a patient diagnosed with AECOPD, (b) contacting the blood sample with an anti-cardiac troponin antibody and an anti-copeptin antibody, (c) detecting binding of cardiac troponin to the anti-cardiac troponin antibody and binding of copeptin to the anti-copeptin antibody, (d) quantifying the amounts of cardiac troponin and copeptin in the blood sample, wherein increased levels of cardiac troponin and copeptin in the blood sample as compared to normal levels of cardiac troponin and copeptin indicate an increased risk of short-term mortality in the patient.

In one aspect of the above method, the CURB-65 score of the patient is determined.

In another aspect of the above method, the patient comprises a CURB-65 score of between 3 and 5.

In another aspect, the patient is hospitalized for AECOPD and short-term mortality is death of the patient within 30 days of hospitalization.

In another aspect, the patient having an increased risk of short-term mortality is diagnosed with acute myocardial injury (AMI).

In yet another aspect of the above method, the AMI in the patient is treated.

In another aspect, an increased risk of short-term mortality in the patient is indicated when the level of cardiac troponin in the patient is at least 2-fold greater than the normal level of cardiac troponin and the level of copeptin in the patient is at least 2-fold greater than the normal level of copeptin.

In still yet another aspect of the above method, the blood sample is plasma.

In a further aspect, the cardiac troponin is cardiac troponin I (cTnI).

In another aspect, binding of cardiac troponin to the anti-cardiac troponin antibody is detected with a high-sensitivity cardiac troponin assay (hs-cTn).

### BRIEF DESCRIPTION OF THE SEVERAL VIEWS OF THE DRAWING(S)

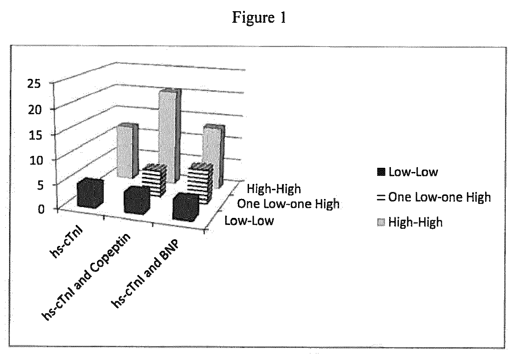

Figure 1 is a graph which illustrates 30-day mortality in AECOPD patients depending on hs-cTnI, copeptin and/or BNP abnormality.

### DETAILED DESCRIPTION OF THE INVENTION

Embodiments of the present disclosure relate to methods for detecting cardiac troponin (cTn) and copeptin proteins in samples obtained from COPD patients, particularly for determining risk of short-term mortality in patents having an acute exacerbation of COPD (AECOPD).

### 1. Definitions

Before the embodiments of the present disclosure are described, it is to be understood that this invention is not limited to particular embodiments described, as such may, of course, vary. It is also to be understood that the terminology used herein is for the purpose of describing particular embodiments only, and is not intended to be limiting.

"Acute myocardial infarction" or "AMI" refers to the interruption of blood supply to a part of the heart, causing restriction in blood supply ("ischemia"), lack of oxygen, and cell death ("necrosis"). This may result in damage or death of heart muscle tissue (myocardium). Thus, "myocardial necrosis" refers to the death of heart cells. AMI may be classified as ST elevation myocardial infarction (STEMI), diagnosed by elevation of the ST segment of the electrocardiogram, and non-ST elevation myocardial infarction (non-STEMI), diagnosed by absence of such electrocardiographic changes. STEMI may be treated with fibrinolysis, thrombolysis or percutaneous coronary intervention (PCI). Non-STEMI may be managed with medication, although PCI is often performed during hospital admission.

As used herein, "admission" refers to the formal acceptance by a hospital or other health care facility of a subject who is to be provided with medical treatment. In particular, "admission" will be associated with an accurate time at which the subject is accepted for medical treatment.

"Antibody" and "antibodies" refer to monoclonal antibodies, multispecific antibodies, bifunctional antibodies, human antibodies, humanized antibodies (fully or partially humanized), animal antibodies (such as, but not limited to, antibodies obtained or derived from a bird (for example, a duck or a goose), a shark, a whale, and a mammal, including a non-primate (for example, a cow, a pig, a camel, a llama, a horse, a goat, a rabbit, a sheep, a hamster, a guinea pig, a cat, a dog, a rat, a mouse, etc.) or a non-human primate (for example, a monkey, a chimpanzee, etc.), recombinant antibodies, chimeric antibodies, single-chain Fvs ("scFv"), single chain antibodies, single domain antibodies, Fab fragments, F(ab') fragments, F(ab')₂ fragments, disulfide-linked Fvs ("sdFv"), and anti-idiotypic ("anti-Id") antibodies, dual-domain antibodies, dual variable domain (DVD) or triple variable domain (TVD) antibodies (see, e.g., Wu et al., Nature Biotechnology, 25(11): 1290-1297 (2007), and International Patent Application Publication No. WO 2001/058956)), and functionally active epitope-binding fragments of any of the above. The term "bifunctional antibody," as used herein, refers to an antibody that comprises a first arm having a specificity for one antigenic site and a second arm having a specificity for a different antigenic site, i.e., the bifunctional antibodies have a dual specificity.

The terms "antibody fragment" and "antibody fragments" refer to a portion of an intact antibody comprising the antigen-binding site or variable region. The portion does not include the constant heavy chain domains (i.e., CH2, CH3 or CH4, depending on the antibody isotype) of the Fc region of the intact antibody. Examples of antibody fragments include, but are not limited to, Fab fragments, Fab' fragments, Fab'-SH fragments, F(ab')₂ fragments, Fd fragments, Fv fragments, diabodies, single-chain Fv (scFv) molecules, single-chain polypeptides containing only one light chain variable domain, single-chain polypeptides containing the three CDRs of the light-chain variable domain, single-chain polypeptides containing only one heavy chain variable region, and single-chain polypeptides containing the three CDRs of the heavy chain variable region.

As used herein, the term "biomarker" refers to a measurable substance, the detection of which indicates a particular disease or risk of acquiring a particular disease. A "biomarker" may indicate a change in expression or state of the measurable substance that correlates with the prognosis of a disease. A "biomarker" may be a protein or peptide. A "biomarker" may be measured in a bodily fluid such as plasma. In the context of the method described herein, a "biomarker" can be a troponin (e.g., cardiac troponin) and/or copeptin.

"Chronic obstructive pulmonary disease" (COPD) refers to a chronic inflammatory lung disease characterized by limitation of airflow in the airway that is not fully reversible. Symptoms include, but are not limited to, breathing difficulty, cough, sputum production, and wheezing. COPD is caused by long-term exposure to irritating gases or particulate matter, most often from cigarette smoke. Individuals suffering from COPD have increased risk of developing heart disease, lung cancer, and a variety of other conditions. Emphysema and chronic bronchitis are the two most common conditions that contribute to COPD. Chronic bronchitis is inflammation of the lining of the bronchial tubes, which carry air to and from the air sacs (alveoli) of the lungs, and characterized by daily cough and sputum production. Emphysema is a condition in which the air sacs (alveoli) at the end of the smallest air passages (bronchioles) of the lungs are destroyed as a result of damaging exposure.

An acute exacerbation of chronic obstructive pulmonary disease (AECOPD) is a clinical diagnosis made when a patient with COPD experiences a sustained (e.g., 24-48 hours) increase in cough, sputum production, and/or dyspnea. AECOPD has clinical consequences ranging from a self-limited illness to progressive respiratory failure requiring mechanical ventilation (see, e.g., O'Donnell et al., Thorax, 61:354-361 (2006); Bach et al., Ann Intern Med, 134:600-620 (2001); Ball, P., Chest, 108:43S-52S (1995); Celli, B.R. and Barnes P.J., Eur Respir J, 29:1224-1238 (2007); and MacIntyre, N., & Huang, Y. C., Proceedings of the American Thoracic Society, 5(4), 530-535 (2008)). Although bacterial infections are the most common causes of AECOPD, viral infections and environmental stresses also are implicated. AECOPD episodes can be triggered or complicated by other comorbidities, such as heart disease, other lung diseases (e.g., pulmonary emboli, aspiration, pneumothorax), or systemic processes.

The term "copeptin," as used herein, refers to a 39-amino acid glycopeptide that comprises the C-terminal portion of the arginine vasopressin (AVP) precursor protein. AVP, also known as antidiuretic hormone (ADH), is involved in multiple cardiovascular and renal pathways. Preanalytic instability of AVP has limited its clinical applications; thus, copeptin has been identified as a stable and sensitive surrogate marker for AVP release. Serum copeptin is glycosylated and contains a sugar moiety.

As used herein, "diagnosis" and similar terms refer to the identification of a particular disease.

The term "dyspnea," as used herein, refers to shortness of breath and the feeling or feelings associated with impaired breathing.

"Label" and "detectable label" mean a moiety attached, directly or indirectly, to an analyte-binding molecule (e.g., antibody or analyte-reactive fragment thereof) or an analyte to render the reaction between the analyte-binding molecule (e.g., antibody or analyte-reactive fragment thereof) and the analyte detectable, and the an analyte-binding molecule (e.g., antibody or analyte-reactive fragment thereof) or analyte so labeled is referred to as "detectably-labeled." A label can produce a signal that is detectable, e.g., by visual or instrumental means. In this aspect, a label can be any signal-generating moiety, and sometimes is referred to herein as a reporter group. As used herein, the label (or signal-generating moiety) produces a measurable signal which is detectable by external means, e.g., by the measurement of electromagnetic radiation, and, depending on the system employed, the level of signal can vary to the extent the label is in the environment of the solid support, e.g., an electrode, microparticle or bead.

The term "low risk," as used herein, is defined as less than or equal to a 10% chance, preferably less than a 5% chance, and more preferably less than a 2% chance of an AECOPD patient dying within 30 days of hospital admission. The term "moderate risk," as used herein, is defined as greater than a 10% and less than a 30% chance of an AECOPD patient dying within 30 days of hospital admission. The term "high risk," as used herein, is defined as greater than a 25% chance, preferably greater than or equal to a 30% chance, and more preferably greater than a 35% chance of an AECOPD patient dying within 30 days of hospital admission. It should be noted that the ranges and cutoff points recited herein in connection with the terms "low risk," "moderate risk," and "high risk" may vary depending upon the specific study utilized in order to gather the relevant data in connection with risk assessment. Further, it should be noted that these cutoff points relate to event risk and not relative risk.

"Predetermined cutoff," "cutoff," "predetermined level," and "reference level" as used herein refer to an assay cutoff value that is used to assess diagnostic, prognostic, or therapeutic efficacy results by comparing the assay results against the predetermined cutoff/level, where the predetermined cutoff/level already has been linked or associated with various clinical parameters (e.g., presence of disease, stage of disease, severity of disease, progression, non-progression, or improvement of disease, etc.). The disclosure provides exemplary predetermined levels and reference levels. However, it is well-known that cutoff values may vary depending on the nature of the immunoassay (e.g., antibodies employed, reaction conditions, sample purity, etc.). It further is well within the ordinary skill of one in the art to adapt the disclosure herein for other immunoassays to obtain immunoassay-specific cutoff values for those other immunoassays based on the description provided by this disclosure. Whereas the precise value of the predetermined cutoff/level may vary between assays, the correlations as described herein should be generally applicable.

"Risk assessment," "risk classification," "risk identification," or "risk stratification" of subjects (e.g., patients) as used herein refers to the evaluation of factors including biomarkers, to predict the risk of occurrence of future events including disease onset or disease progression, so that treatment decisions regarding the subject may be made on a more informed basis.

"Sample," "biological sample," "test sample," "specimen," "sample from a subject," and "patient sample" as used herein may be used interchangeable and may be a sample of blood, tissue, urine, serum, plasma, amniotic fluid, cerebrospinal fluid, placental cells or tissue, endothelial cells, leukocytes, or monocytes. The sample can be used directly as obtained from a patient or can be pre-treated, such as by filtration, distillation, extraction, concentration, centrifugation, inactivation of interfering components, addition of reagents, and the like, to modify the character of the sample in some manner as discussed herein or otherwise as is known in the art.

Any cell type, tissue, or bodily fluid may be utilized to obtain a sample. Such cell types, tissues, and fluid may include sections of tissues such as biopsy and autopsy samples, frozen sections taken for histologic purposes, blood (such as whole blood), plasma, serum, sputum, stool, tears, mucus, saliva, bronchoalveolar lavage (BAL) fluid, hair, skin, red blood cells, platelets, interstitial fluid, ocular lens fluid, cerebral spinal fluid, sweat, nasal fluid, synovial fluid, menses, amniotic fluid, semen, etc. Cell types and tissues may also include lymph fluid, ascetic fluid, gynecological fluid, urine, peritoneal fluid, cerebrospinal fluid, a fluid collected by vaginal rinsing, or a fluid collected by vaginal flushing. A tissue or cell type may be provided by removing a sample of cells from an animal, but can also be accomplished by using previously isolated cells (e.g., isolated by another person, at another time, and/or for another purpose). Archival tissues, such as those having treatment or outcome history, may also be used. Protein or nucleotide isolation and/or purification may not be necessary.

Methods well-known in the art for collecting, handling and processing urine, blood, serum and plasma, and other body fluids, are used in the practice of the present disclosure. The test sample can comprise further moieties in addition to the analyte of interest, such as antibodies, antigens, haptens, hormones, drugs, enzymes, receptors, proteins, peptides, polypeptides, oligonucleotides or polynucleotides. For example, the sample can be a whole blood sample obtained from a subject. It can be necessary or desired that a test sample, particularly whole blood, be treated prior to immunoassay as described herein, e.g., with a pretreatment reagent. Even in cases where pretreatment is not necessary (e.g., most urine samples, a pre-processed archived sample, etc.), pretreatment of the sample is an option that can be performed for mere convenience (e.g., as part of a protocol on a commercial platform). The sample may be used directly as obtained from the subject or following pretreatment to modify a characteristic of the sample. Pretreatment may include extraction, concentration, inactivation of interfering components, and/or the addition of reagents.

As used herein, the terms "prognosis," "prognosticate," and related terms refer to the description of the likely outcome of a particular condition, e.g., AECOPD. For example, in a subject with suspected AECOPD, measurement of plasma cardiac troponin and copeptin concentrations enables determination of risk of short-term mortality, because plasma cTn and copeptin concentrations correlate with an increased risk of short-term mortality in AECOPD.

As used herein, the term "short-term mortality" refers to the death of a subject within 30 days of hospitalization for AECOPD.

As used herein, the terms "subject" and "patient" are used interchangeably irrespective of whether the subject has or is currently undergoing any form of treatment. As used herein, the terms "subject" and "subjects" refer to any vertebrate, including, but not limited to, a mammal (e.g., cow, pig, camel, llama, horse, goat, rabbit, sheep, hamsters, guinea pig, cat, dog, rat, and mouse, a non-human primate (for example, a monkey, such as a cynomolgous monkey, chimpanzee, etc.) and a human). Preferably, the subject is a human.

The terms "treat," "treated," or "treating," as used herein, refer to a therapeutic method wherein the object is to slow down (lessen) an undesired physiological condition, disorder or disease, or to obtain beneficial or desired clinical results. For the purposes of this invention, beneficial or desired clinical results include, but are not limited to, alleviation of symptoms; diminishment of the extent of the condition, disorder or disease; stabilization (i.e., not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment also includes prolonging survival as compared to expected survival if not receiving treatment.

The term "troponin" refers to a complex of three regulatory proteins (i.e., troponin C, troponin I, and troponin T) that is located on the thin filament of the muscle contractile apparatus and plays an integral role in contraction of skeletal and cardiac muscle. Troponin I is a muscle protein which may be used in the determination of myocardial damage subsequent to or during, for example, a myocardial infarction. The other two subunits (i.e., T and C) also are immobilized on the thin myofilaments with troponin I in cardiac and skeletal muscle tissue. The cardiac forms of troponin C (cTnT) and troponin I (cTnI) are cardiac regulatory proteins that control the calcium-mediated interaction between actin and myosin. While expression of cTnI has not been identified outside of myocardium, cTnT is expressed to small extent in skeletal muscle (see, e.g., Bodor et al., Clin. Chem., 41:1710 (1995); and Ricchiuti et al., Clin. Chem., 44:1919(1998)).

### 2. Detection of Cardiac Troponin and Copeptin

In one embodiment, the invention provides a method of detecting cardiac troponin (cTn) and copeptin proteins in a sample obtained from a patient diagnosed with chronic obstructive pulmonary disease (COPD). The method comprises (a) obtaining a blood sample from a patient diagnosed with COPD, and (b) detecting whether cardiac troponin and copeptin are present in the blood sample by contacting the blood sample with an anti-cardiac troponin antibody and an anti-copeptin antibody.

Any suitable sample can be obtained from the patient. As defined above, suitable samples include, for example, blood, serum, urine, saliva, lung tissue, pleural fluid, and cardiac tissue. Preferably, the sample is blood. More preferably, the sample is plasma. Plasma may be obtained by anti-coagulating blood with EDTA, sodium heparin, lithium heparin, sodium citrate, or sodium oxalate. Alternatively, the sample obtained from the patient is serum. In another embodiment, the sample can be whole blood.

A "cardiac troponin" is a troponin that is present in heart tissue and absent or present in undetectable levels in non-cardiac tissues. The method described herein can detect any cardiac troponin expressed in humans, which includes troponin T (cTnT) and troponin I (cTnI). Troponin T has a molecular weight of about 37,000 Da. The troponin T isoform that is found in cardiac tissue (cTnT) is sufficiently divergent from skeletal muscle TnT to allow for the production of antibodies that distinguish both TnT isoforms. cTnT is considered a marker of acute myocardial damage (see, e.g., Katus et al., J. Mol. Cell. Cardiol., 21: 1349-1353 (1989); Hamm et al., N. Engl. J. Med., 327: 146-150 (1992); Ohman et al., N. Engl. J. Med., 335: 1333-1341 (1996); Christenson, Clin. Chem., 44: 494-501(1998); and U.S. Patent 6,376,206).

Troponin I (TnI) is a 25 KDa inhibitory element of the troponin complex found in muscle tissue. TnI binds to actin in the absence of Ca²⁺, inhibiting the ATPase activity of actomyosin. The TnI isoform expressed in cardiac tissue (cTnI) is 40% divergent from skeletal muscle TnI, allowing both isoforms to be immunologically distinguished. The normal plasma concentration of cTnl is <0.1 ng/ml (4 pM). cTnl is released into the bloodstream following cardiac cell death; thus, the plasma cTnl concentration is elevated in patients with acute myocardial infarction (Benamer et al., Am. J. Cardiol., 82: 845-850 (1998)).

As discussed above, copeptin is a glycosylated 39-amino acid peptide with a leucine-rich core segment that shares the same precursor peptide as arginine vasopressin (AVP). The 164-amino acid long preprovasopressin consists of a signal peptide, AVP, neurophysin II, and copeptin (Land et al., Nature, 295: 299-303 (1982)). Thus, copeptin is the C-terminal part of pro-AVP (CT-proAVP) and is released together with AVP during precursor processing. In contrast to AVP, copeptin is very stable in the serum or plasma at room temperature and is used as a sensitive surrogate marker for AVP release. In this respect, copeptin measurement has been used in the clinical diagnosis of cardiovascular diseases such as heart failure, myocardial infarction, and stroke, and as a biomarker for renal function (see, e.g., Morgenthaler, N.G., Congestive Heart Failure, 16 (Suppl. 1): S37-44 (2010)).

Cardiac troponin and copeptin can be detected in the blood sample using any suitable method known in the art for detecting proteins in biological samples. Preferably, the methods described herein comprise detecting whether cardiac troponin and copeptin are present in the blood sample by contacting the blood sample with an anti-cardiac troponin antibody and an anti-copeptin antibody or fragments (e.g., antigen-binding fragments) thereof. Antibodies which bind to cardiac troponins, and components thereof, are known in the art (see, e.g., U.S. Patents 8,030,026 and 8,835,120; U.S. Patent Application Publication 2007/0172888; and Krintus et al., Clin. Chem. Lab Med.; 52(11):1657-65 (2014)). Anti-cardiac troponin antibodies also are commercially available from sources such as, for example, Abbott Laboratories (Abbott Park, IL), Roche Diagnostics USA (Indianapolis, IN), and ThermoFisher Scientific, Inc. (Waltham, MA). Likewise, antibodies which bind copeptin, and components thereof, are known in the art (see, e.g., U.S. Patent 7,807,397 and Morgenthaler et al., Clin. Chem., 52(1): 112-119 (2006)). Anti-copeptin antibodies also are commercially available from sources such as, for example, ThermoFisher Scientific, Inc. (Waltham, MA) and R&D Systems, Inc., Minneapolis, MN).

Cardiac troponin and copeptin can be detected in the blood sample using whole antibodies, as described herein, or antibody fragments, as described herein. In embodiments where the cardiac troponin and copeptin are detected with antibody fragments, the fragment can be of any size so long as the fragment binds to cardiac troponin and/or copeptin. In this respect, a fragment of an anti-cardiac troponin antibody and/or an anti-copeptin antibody comprises a heavy chain polypeptide and light chain polypeptide, each of which desirably comprises between about 5 and 18 (e.g., about 5, 6, 7, 8, 9, 10, 11, 12, 13, 14, 15, 16, 17, 18, or a range defined by any two of the foregoing values) amino acids.

Detecting binding of the anti-cardiac troponin antibody (or antigen-binding fragment thereof) and the anti-copeptin antibody (or antigen-binding fragment thereof) to cardiac troponin and copeptin, respectively, can be performed using any suitable assay known in the art. Examples of suitable assays include, but are not limited to, immunoassay, such as sandwich immunoassay (e.g., monoclonal-polyclonal sandwich immunoassays, including enzyme detection (enzyme immunoassay (EIA) or enzyme-linked immunosorbent assay (ELISA), competitive inhibition immunoassay (e.g., forward and reverse), enzyme multiplied immunoassay technique (EMIT), a competitive binding assay, bioluminescence resonance energy transfer (BRET), one-step antibody detection assay, homogeneous assay, heterogeneous assay, capture on the fly assay, and the like.

The immunoassay methods of the present disclosure can be carried out in any of a wide variety of formats, descriptions of which are provided in, e.g., Asai, ed., Methods in Cell Biology Volume 37: Antibodies In Cell Biology, Academic Press, Inc. New York (1993), and Stites & Terr, eds., Basic and Clinical Immunology 7th Edition, (1991). A typical heterogeneous sandwich immunoassay employs a solid phase (as a solid support) to which is bound a first (capture) antibody reactive with at least one epitope on an analyte of interest that is an antigen. A second (detection) antibody is also reactive with at least one epitope on the analyte of interest that is an antigen. The second antibody may be conjugated to a detectable label that provides a signal that is measured after the detection antibody binds to the captured analyte. When a test sample containing the analyte contacts the first antibody, the first antibody captures the analyte of interest. The analyte of interest is contacted with the second antibody resulting in the formation of an immunodetection complex consisting of the first antibody, analyte of interest and second antibody, and the complex is bound to the solid phase. The signal generated by the second (detection) antibody is proportional to the concentration of the analyte of interest as determined by the rate of formation (k1) of the immunodetection complex versus the rate of dissociation of the immunodetection complex (k2). Heterophilic endogenous antibodies and any autoantibodies, which if present are unpredictable as to exactly where on the analyte of interest they will bind, can substantially interfere with binding of the first and/or second antibody, and thus with the resulting signal.

In one embodiment, the binding of cardiac troponin to the anti-cardiac troponin antibody is detected with a high-sensitivity cardiac troponin assay (hs-cTn). By "high-sensitivity" is meant that the assay exhibits higher sensitivity for a cardiac troponin than other assays known in the art or commercially available. High-sensitivity cardiac troponin assays have been described in the art (see, e.g., Krintus et al., *supra*; and U.S. Patent 8,835,120) and are commercially available from a variety of sources (e.g., ARCHITECT® high-sensitivity troponin I assay (Abbott Diagnostics, Lake Forest, IL; Elecsys troponin T high-sensitive assay (Roche Diagnostics GmbH, Mannheim, Germany); AccuTnI+3 troponin I assay (Beckman Coulter, Brea, CA), and assays available from SINGULEX®, Alameda, CA).

Assays used to detect cardiac troponin (cTn) and copeptin proteins in a sample obtained from a patient diagnosed with chronic obstructive pulmonary disease (COPD) can be adapted for use in a variety of automated and semi-automated systems (including those wherein the solid phase comprises a microparticle), such as those described in, e.g., in U.S. Patents 5,089,424 and 5,006,309, and commercially marketed, e.g., by Abbott Laboratories (Abbott Park, Ill.) as ARCHITECT®.

Differences between an automated or semi-automated system as compared to a non-automated system (e.g., ELISA) include, for example, the substrate to which the first specific binding partner (e.g., an anti-analyte, monoclonal/polyclonal antibody (or a fragment thereof, a variant thereof, or a fragment of a variant thereof) or an anti-analyte DVD-Ig (or a fragment thereof, a variant thereof, or a fragment of a variant thereof) is attached, as well as the length and timing of the capture, detection and/or any optional wash steps. While a non-automated format, such as an ELISA, may require a relatively longer incubation time with sample and capture reagent (e.g., about two hours), an automated or semi-automated format (e.g., ARCHITECT®) may have a relatively shorter incubation time (e.g., approximately 18 minutes for ARCHITECT®). Similarly, while a non-automated format, such as an ELISA, may incubate a detection antibody, such as the conjugate reagent, for a relatively longer incubation time (e.g., about two hours), an automated or semi-automated format (e.g., ARCHITECT®) may have a relatively shorter incubation time (e.g., approximately four minutes for the ARCHITECT®).

Other platforms available from Abbott Laboratories that can be used in connection with the methods described herein include, but are not limited to, AxSYM®, IMx® (see, e.g., U.S. Patent 5,294,404), PRISM®, EIA (bead), and Quantum™ II, as well as other platforms. Additionally, the methods described herein can be performed in other formats, for example, on electrochemical or other hand-held or point-of-care assay systems (e.g., i-STAT®, Abbott Laboratories) or an electrochemical immunoassay system that performs sandwich immunoassays. Immunosensors and their methods of manufacture and operation in single-use test devices are described in, for example, U.S. Patent 5,063,081, and U.S. Patent Application Publication Nos. 2003/0170881, 2004/0018577, 2005/0054078, and 2006/0160164.

Other assay formats which may be used in connection with the method described herein include, for example, a rapid test, a Western blot, as well as the use of paramagnetic particles in, for example, an ARCHITECT® assay (see Frank Quinn, The Immunoassay Handbook, Second edition, edited by David Wild, pp. 363-367 (2001)), and other appropriate formats known to those of ordinary skill in the art.

The elements of the assays described above can also be used in the form of a kit. The kit may also comprise one or more containers (e.g., vials, bottles, or strips) comprising the assay components and reagents needed for performing the assay (e.g., washing, processing, and indicator reagents).

Methods other than immunoassay can be used to detect cardiac troponin and copeptin in accordance with the inventive method. In this regard, any method that can detect or quantify biomarkers in a sample can be used in the methods described herein. Such methods include physical and molecular biology methods in addition to immunological methods. For example, suitable physical methods include mass spectrometric methods, fluorescence resonance energy transfer (FRET) assays, chromatographic assays, and dye-detection assays. Suitable molecular biology methods include, but are not limited to, Northern or Southern blot hybridization, nucleic acid dot- or slot-blot hybridization, *in situ* hybridization, nucleic acid chip assays, PCR, reverse transcriptase PCR (RT-PCR), or real time PCR (e.g., taq-man PCR). Other methods to detect biomarkers include, e.g., nuclear magnetic resonance (NMR), fluorometry, colorimetry, radiometry, luminometry, or other spectrometric methods, plasmon-resonance (e.g. BIACORE), and one- or two-dimensional gel electrophoresis.

### 3. Determining Increased Risk of Short-Term Mortality in AECOPD

In another embodiment, the invention provides a method of determining risk of short-term mortality in a patient having an acute exacerbation of COPD (AECOPD), which method comprises: (a) obtaining a blood sample from a patient diagnosed with AECOPD, (b) contacting the blood sample with an anti-cardiac troponin antibody (or antigen-binding fragment thereof) and an anti-copeptin antibody (or antigen-binding fragment thereof), (c) detecting binding of cardiac troponin to the anti-cardiac troponin antibody and binding of copeptin to the anti-copeptin antibody, (d) quantifying the amounts of cardiac troponin and copeptin in the blood sample, wherein increased levels of cardiac troponin and copeptin in the blood sample as compared to normal levels of cardiac troponin and copeptin indicate an increased risk of short-term mortality in the patient. Descriptions of the blood sample, anti-cardiac troponin antibody and fragments thereof, anti-copeptin antibody and fragments thereof, methods for detecting antibody binding, and components thereof, set forth above in connection with the method of detecting cardiac troponin and copeptin also are applicable to the aforementioned method of determining risk of short-term mortality in AECOPD.

Following detecting binding of cardiac troponin to the anti-cardiac troponin antibody and binding of copeptin to the anti-copeptin antibody, the method comprises quantifying the amounts of cardiac troponin and copeptin in the blood sample. Any suitable method for quantifying antibody-antigen binding can be used in the methods described herein, a variety of which are known in the art. Typically, quantification of antibody-antigen binding is achieved by measuring a signal produced by a detectable label on the antibody or antigen, such as a radio- or fluorescence-label, and comparing the measured signal to either a standard curve for the protein(s) of interest (e.g., cTn and copeptin) or by comparison to a reference standard for each protein. The reference standard may comprise anti-idiotypic antibodies, or a derivatized cardiac troponin and/or copeptin (e.g., derivatized with a polyethylene glycol).

A moiety on the label may not be detectable itself, but may become detectable upon reaction with yet another moiety (e.g., a secondary detectable label). For example, enzymes can be employed to produce a signal and/or to amplify a signal. As another example, the moiety can be a so-called quencher or an entity upon which a quencher acts. Use of the term "detectably-labeled" is intended to encompass these, and other means, of such labeling.

The detectable label can be any signal-producing substance known in the art, including, for example, an enzyme (e.g., horseradish peroxidase, alkaline phosphatase, alkaline peroxidase, glucose 6-phosphate dehydrogenase, and the like), a chromophore or chromogen (e.g., dyes that absorb light in the ultraviolet or visible region), a phosphor, a fluorescer, a fluorophor (e.g., fluorescent proteins such as green fluorescent protein, yellow fluorescent protein, red fluorescent protein, cyan fluorescent protein); a fluorescent label (e.g., 5-fluorescein, 6-carboxyfluorescein, 3'6-carboxyfluorescein, 5(6)-carboxyfluorescein, 6-hexachloro-fluorescein, 6-tetrachlorofluorescein, fluorescein isothiocyanate, and the like)), rhodamine, quantum dots (e.g., zinc sulfide-capped cadmium selenide), a thermometric label, an immuno-polymerase chain reaction label; a phycobilin (e.g., phycoerythrin, R-Phycoerythrin, B-Phycoerythrin); biotin/avidin; a Xanthene derivative (e.g., fluorescein, rhodamine, Oregon green, eosin, Texas red); a cyanine derivative (e.g., cyanine, Cy dyes, indocarbocyanine, oxacarbocyanine, thiacarbocyanine, merocyanine); a naphthalene derivative (e.g., dansyl and prodan derivatives); a coumarin derivative; a oxadiazole derivative e.g., (pyridyloxazole, nitrobenzoxadiazole, benzoxadiazole); a Pyrene derivative (e.g., cascade blue); an oxazine derivative (e.g., Nile red, Nile blue, cresyl violet, oxazine 170); an acridine derivative (e.g., proflavin, acridine orange, acridine yellow); an arylmethine derivative (e.g., auramine, crystal violet, malachite green); a tetrapyrrole derivative (e.g., porphin, phtalocyanine, bilirubin)), a luminophore, a chemiluminescent compound (e.g., acridinium esters, thioesters, or sulfonamides; luminol, isoluminol, phenanthridinium esters, and the like), a radioactive compound (e.g., such as ³H, ¹²⁵I, ³⁵S, ¹⁴C, ³²P, and ³³P), and the like.

An acridinium compound can be used as a detectable label in a homogeneous chemiluminescent assay (see, e.g., Adamczyk et al., Bioorg. Med. Chem. Lett., 16: 1324-1328 (2006); Adamczyk et al., Bioorg. Med. Chem. Lett., 4: 2313-2317 (2004); Adamczyk et al., Biorg. Med. Chem. Lett., 14: 3917-3921 (2004); and Adamczyk et al., Org. Lett., 5: 3779-3782 (2003)). The acridinium compound can comprise at least one acridinium-9-carboxamide, at least one acridinium-9-carboxylate aryl ester, or any combination thereof. If the detectable label comprises at least one acridinium compound, the method also can comprise the use of a source of hydrogen peroxide, such as a buffer, solution, and/or at least one basic solution.

In one embodiment, the detectable label can be a phycobilin (e.g., phycoerythrin, R-Phycoerythrin, B-Phycoerythrin). R-Phycoerythrin, or PE, is useful as a fluorescence-based indicator for labeling analyte-binding molecules or other molecules in a variety of applications. R-Phycoerythrin absorbs strongly at about 566 nm with secondary peaks at 496 and 545 nm and emits strongly at 575 nm. R-Phycoerythrin is among the brightest known fluorescent dyes (see, e.g., Hayes, M. (ed.), Marine Bioactive Compounds: sources, Characterization and Applications, Springer (2012))

Detectable labels, labeling procedures, and detection of labels are described in detail in, for example, Polak and Van Noorden, Introduction to Immunocytochemistry, 2nd ed., Springer Verlag, N.Y. (1997), and in Haugland, Handbook of Fluorescent Probes and Research Chemicals (1996), which is a combined handbook and catalogue published by Molecular Probes, Inc., Eugene, Oregon.

In another embodiment, the method of determining risk of short-term mortality in AECOPD also involves comparing the levels of cardiac troponin and copeptin in a patient sample with a predetermined value. The predetermined value can take a variety of forms. For example, the predetermined value can be single cut-off value, such as a median or mean. The predetermined value can be established based upon comparative groups, such as where the risk of short-term mortality in one defined group is double the risk in another defined group. In yet another alternative, the predetermined value can be a range, for example, where the tested population is divided equally (or unequally) into groups, such as-a low-risk group, a medium-risk group, and a high-risk group, or into quadrants, the lowest quadrant being individuals with the lowest risk and the highest quadrant being individuals with the highest risk.

The predetermined value can depend upon the particular population selected. For example, an apparently healthy population will have a different normal range of biomarker expression levels than will a population comprised of AECOPD patients. In another embodiment, a population comprised of COPD patients (without acute exacerbation) will have a different range of biomarker expression levels than will a population of AECOPD patients. Accordingly, the predetermined values selected may take into account the category in which an individual falls. Appropriate ranges and categories can be selected by those of ordinary skill in the art using routine methods. The level of a particular biomarker protein (e.g., cardiac troponin and copeptin) may be considered "elevated" if the antibody level measured is above a predetermined threshold level. In one embodiment, such a threshold level can be set to the 90 th-percentile or to the 95th-percentile of a healthy control population. Preferably, the threshold level is established at the 95th-percentile of a healthy control population. In one embodiment, an increased risk of short-term mortality in the patient is indicated when the level of cardiac troponin in the patient is at least 2-fold greater (e.g., 2, 5, 10, 15, 20, 25, 30, 35, 40, 45, 50-fold, or greater) than a predetermined normal level of cardiac troponin and the level of copeptin in the patient is at least 2-fold greater (e.g., 2, 5, 10, 15, 20, 25, 30, 35, 40, 45, 50-fold, or greater) than a predetermined normal level of copeptin.

If an AECOPD patient is determined to have an increased risk of short-term mortality in accordance with the methods described herein, the AECOPD patient also may be diagnosed with acute myocardial infarction (AMI). AMI can be diagnosed using any suitable method known in the art, some of which involve the detection of cardiac troponin and/or copeptin (see, e.g., Möckel, M. and Searle, J., Curr. Atheroscler. Rep., 16(7): 421 (2014); Daubert, M.A., and Jeremias, A., Vasc. Health Risk Management, 6:691-699 (2010); Mahajan et al., Circulation, 124: 2350-2354 (2011); and Maisel et al., J Am. Coll. Cardiol., 62(2):150-160 (2013)). Other methods known in the art for diagnosing AMI also can be used in connection with the methods described herein, including, for example, electrocardiogram (EKG), stress test, angiogram, and/or echocardiogram. In embodiments where the AECOPD patient also is diagnosed with AMI, the inventive method further comprises treating the AMI in the patient. AMI can be treated using a variety of therapies, which include pharmaceutical, surgical, and rehabilitative strategies. Pharmaceutical treatments for AMI include, for example, antiplatelet agents (e.g., aspirin), supplemental oxygen, nitrates (e.g., nitroglycerin), analgesics (e.g., morphine), beta blockers (e.g., metoprolol, atenolol, carvedilol), unfractionated heparin, low molecular weight heparin, warfarin, fibrinolytics, angiotensin-converting enzyme (ACE) inhibitors, glycoprotein IIb/IIIa inhibitors, statins, and aldosterone antagonists (see, e.g., Maxwell, S., Br. J. Clin. Pharmacol., 48(3): 284-298 (1999)). Surgical procedures for treating AMI include, for example, percutaneous coronary intervention, surgical revascularization (e.g., coronary artery bypass grafting), and implantable cardiac defibrillators. Treatment strategies for myocardial infarction are described in detail in, for example, O'Gara et al., 2013 ACCF/AHA Guideline for the Management of ST-Elevation Myocardial infarction: a report of the American College of Cardiology Foundation/American Heart Association Task Force on Practice Guidelines., Circulation, 127 (4): e362-425 (2013); Modi et al., J. La. State. Med. Soc. Jun., 153(6):284-90 (2001); and Danchin et al., Am. Fam. Physician, 68(3): 519-52 (2003).

In another embodiment, the method for determining risk of short-term mortality in AECOPD can be performed in conjunction with other criteria or tools for prognosticating or stratifying mortality risk. Such measures include, for example, the BODE (**B**ody mass index, airflow **O**bstruction, **D**yspnea, and **E**xercise capacity) index for COPD survival prediction (Celli et al., N. Engl. J. Med., 350(10): 1005-12 (2004)); the GOLD (**G**lobal Initiative for Chronic **O**bstructive **L**ung **D**isease) staging system (Global Strategy for the Diagnosis, Management and Prevention of COPD, Global Initiative for Chronic Obstructive Lung Disease (GOLD) 2016), the "2008 Score" (Roche et al., Respiratory Research, 15: 99 (2014), the FEV1/FVC ratio (Swanney et al., Thorax, 63(12): 1046-1051 (2008)), and the CURB-65 score.

In one embodiment, the methods described herein further comprise determining the CURB-65 score of the patient. CURB-65 is a six-point score that has been validated for predicting mortality in community acquired pneumonia (Lim et al., *supra*). As discussed above, the CURB-65 score also has been associated with in-hospital and 30-day mortality after AECOPD in both retrospective and prospective studies (see, e.g., Chang et al., Internal Medicine Journal, 37(4): 236-241 (2007) and Chang et al., Respirology, 16(1): 146-151 (2011)). The score is an acronym for each of the risk factors measured. Each risk factor scores one point, for a maximum score of 5: **C**onfusion of new onset, Blood **U**rea nitrogen greater than 7 mmol/l (19 mg/dL), **R**espiratory rate of 30 breaths per minute or greater, **B**lood pressure less than 90 mmHg systolic or diastolic blood pressure 60 mmHg or less, Age **65** or older. The risk of death at 30-days post hospital admission increases as the CURB-65 score increases, i.e., CURB-65 scores of 0-1 are classified as low risk, a CURB-65 score of 2 is classified as intermediate risk, and CURB-65 scores of 3-5 are classified as high risk of 30-day mortality (Lim et al. *supra*). In one embodiment, an AECOPD patient determined to have an increased risk of short-term mortality using the methods described herein desirably also comprises a CURB-65 score between 3 and 5 (i.e., 3, 4, or 5). As demonstrated in the Examples, the methods described herein improve the prognostic value of the CURB-65 score to predict short-term mortality in AECOPD patients.

The following examples further illustrate the invention but, of course, should not be construed as in any way limiting its scope.

### EXAMPLE 1

This example demonstrates a method of detecting cardiac troponin and copeptin in samples obtained from AECOPD patients.

### Study design and participants

For a sample cohort study, 356 consecutive patients with AECOPD were prospectively enrolled in the Christchurch Breathless Cohort (New Zealand, n = 160), Biomarcoeurs cohort in Lariboisière University Hospital in Paris (France, n = 112), Monastir University Hospital (Tunisia, n = 55), and in Sivas University Hospital (Turkey, n = 29). Inclusion criteria were as follows: adult patients (> 18 years old) presenting to the emergency department (ED) with AECOPD defined by a "change in the patient's baseline dyspnea, cough, and/or sputum that is beyond normal day-to-day variations" (Jenkins et al., Respir. Res., 10: 59 (2009)), and a history of exposure to risk factors such as cigarette smoking. AECOPD was diagnosed by the admitting physician and confirmed by review of hospital records. In the Biomarcoeurs cohort, patients were excluded if one of the following criteria was present: cognitive dysfunction, pregnancy, or patients without insurance affiliation.

Clinical data and routine blood tests were gathered in all patients by admitting physicians upon admission to the ED. No recommendation was made to the attending physician for the management of patients. Decision for intensive care unit (ICU), hospital admission, or discharge from the initial visit to the ED was made by the attending physician in charge who incorporated the patients' requests and hospital capacity into the decision making.

Clinical characteristics of the 356 patients showed a median age of 72 (63-79) years, and 57% were male (Table 1). Prior history of COPD was present in 85% of patients, heart failure was present in 20%, and systemic hypertension was present in 41%. 21% of patients received chronic home oxygen therapy. At admission, the median systolic blood pressure was 140 (123-160) mmHg, median heart rate was 98 (86-112)/min, and median respiratory rate was 26 (22-32)/min.

**Table 1**

| | **Total population** | **Alive at D30** | **Dead at D30** | ***P*** |
|---|---|---|---|---|
| N | 356 | 329 (92.4%) | 27 (7.6%) | |
| **Age, yrs** | **72 [63-79]** | **71 [63**-**79]** | **79 [71**-**82]** | **0.009** |
| Age > 65 yo | 252 (71) | 229 (70) | 23 (85) | 0.087 |
| Male | 203 (57) | 186 (57) | 17(63) | 0.528 |
| **Hospital LOS (days)** | **4 [1-8]** | **5 [3-11]** | **4 [2-20]** | **0.045** |
| BMI, kg/m² | 24 [21-29] | 24 [21-29] | 26 [21-29] | 0.885 |

| | **Total population** | **Alive at D30** | **Dead at D30 *P*** | |
|---|---|---|---|---|
| **Comorbidities** | | | | |
| Previous diapnosis of COPD | 303 (85) | 281 (85) | 22 (82) | 0.581 |
| Hypertension | 145 (41) | 135(41) | 10 (37) | 0.685 |
| Smokers | 125 (35) | 117 (36) | 8 (30) | 0.535 |
| Chronic Heart Failure | 71 (20) | 66 (20) | 5 (19) | 0.847 |
| Diabetes mellitus | 70 (20) | 62(19) | 8 (30) | 0.175 |
| LVEF, % | 55 [47-60] | 55 [48-60] | 50 [48-54] | 0.262 |
| **Chronic treatment** | | | | |
| Antiplatelets | 60 (17) | 55 (17) | 5(19) | 0.810 |
| Beta-Blockers | 21 (6) | 20 (6) | 1 (4) | 0.615 |
| ACE inhibitors | 66 (19) | 59(18) | 7 (26) | 0.304 |
| Statin | 30(8) | 26 (8) | 4 (15) | 0.214 |
| Oxygen @ home | 73 (21) | 66 (20) | 7 (26) | 0.468 |
| | | | | |
| **Clinical signs at admission** | | | | |
| SBP, mmHg | 140 [123-160] | 140 [123-160] | 135 [119-152] | 0.399 |
| DBP, mmHg | 78 [67-88] | 78 [68-88] | 74 [65-87] | 0.358 |
| Heart rate, bpm | 98 [86-112] | 98 [86-112] | 104 [94-113] | 0.070 |
| Respiratory rate, cpm | 26 [22-32] | 26 [22-32] | 26 [22-32] | 0.989 |
| Temperature, °C | 37.0 [36.0-37.01] | 37.0 [36.5-37.0] | 36.5 [36.0-37.0] | 0.558 |
| **Oxygen saturation, %** | **93 [88-96]** | **93 [88-96]** | **89 [83-93]** | **0.007** |
| | | | | |
| **Biology at admission** | | | | |
| eGFR, ml/min/1.73 m² | 66 [53-80] | 67 [53-79] | 59 [38-90] | 0.918 |
| **PaCO2**, **mmHg** | **47 [38-59]** | **46 [38**-**59]** | **59 [45-71]** | **0.024** |
| | | | | |
| **CURB65 categories** | | | | **0.020** |
| Low | 182 (51) | 174 (53) | 8 (30) | |
| Medium | 115 (32) | 105 (32) | 10 (37) | |
| High | 59 (17) | 50 (15) | 9(33) | |

In Table 1, data are expressed as numbers (percentage) or median (interquartile range). LOS hospital: Length Of hospital Stay, BMI: Body mass index, COPD=chronic obstructive pulmonary disease, LVEF=left ventricular ejection fraction, ACE: angiotensine converting enzyme inhibitors, SBP=systolic blood pressure, DBP=diastolic blood pressure, bpm=beats per minute, cpm=counts per minute, Temperature in °C for degree Celsius, eGFR=estimated glomerular filtration rate by modification of diet in renal disease formula, CURB65 score: confusion, urea, respiratory rate, blood pressure and age.

Initial management primarily consisted of inhaled broncho-dilating agents, antibiotics, and systemic corticosteroids (Table 2). The large majority of studied patients (n=286, 80.3%) were hospitalized for more than 24 hours, including 44 patients (12.4%) in an intensive care unit (ICU). The median length of hospital stay was 4 (1-8) days. Twenty seven patients (7.6%) died during the 30-day follow-up period.

**Table 2**

| **ED management** | **Total population** | **Alive at D30** | **Dead at D30** | *P* |
|---|---|---|---|---|
| Systemic corticosteroids | 142 (40) | 130 (40) | 12 (44) | 0.615 |
| Antibiotics | 156 (44) | 142 (43) | 14(52) | 0.382 |
| Bronchodilating agents | 259 (73) | 236 (72) | 23 (85) | 0.131 |
| Non Invasive | | | | |
| Ventilation | 40(11) | 33 (10) | 7 (26) | **0.012** |
| Mechanical Ventilation | 7 (2) | 4 (1) | 3(11) | **0.011** |

### Statistical Analysis

Values described below are expressed as number and percentage or median and first to third quartile. CURB65 scores were used to predict 30-day mortality. Comparisons between groups were performed using a χ² test or Wilcoxon test as appropriate. Biomarker results were assessed as described below.

First, the marginal association of each biomarker of interest with 30-day mortality was studied using the Wilcoxon test. Areas under receiver-operator curves (AUC-ROC) for prediction of 30-day mortality were determined and the optimal threshold value of individual candidate markers was estimated using the "closest top left" method. Second, the association of each biomarker to the primary end point was assessed with adjustment for previously described predictive factors using multiple logistic regression. Biomarkers were considered either continuously or dichotomized according to the threshold obtained from the ROC curves.

Biomarkers remaining significantly associated with 30-day mortality after adjustment reclassification analysis by both net reclassification improvement (NRI) and integrated discrimination index (IDI) was used to evaluate the benefit of the addition of these biomarkers to the CURB-65 score (Pencina et al., Stat. Med.; 27(2): 157-72 (2008); and Cook, N.R., Clin. Chem., 54(1): 17-23 (2008)). A two-sided p-value of 0.05 was considered significant. All analyses were performed using R 2.10.1 statistical software (The R Foundation for Statistical Computing, Vienna, Austria). ROC curve analyses were performed using the package "pROC" and reclassification analyses using "PredictABEL" package.

### CURB-65 Score Measurement

CURB-65 scores were calculated using information collected upon first admission to the ED. The CURB-65 score was low (i.e., 0 to 1) in 51 % of the cohort, intermediate (i.e., 2) in 32% of the cohort, and high (i.e., ≥ 3) in 17% of the cohort. ROC curve analysis yielded an area under the curve (AUC) of 0.67 [0.58 - 0.67] for the prediction of 30-day mortality by the CURB-65 score. AUCs for other individual markers ranged between 0.58 to 0.70.

### Biomarkers Testing

All study blood samples were collected by research coordinators at enrollment (within four hours of admission) into tubes containing ethylenediaminetetra-acetic acid (EDTA) and stored in biobank facility at -80°C for further analysis. Six biomarkers reflecting different pathophysiological pathways were measured: inflammation and/or infection as indicated by c-reactive protein (CRP, Abbott laboratories, Abbott Park, IL, USA) and procalcitonin (PCT, Thermo-Fisher B.R.A.H.M.S. AG, Hennigsdorf, Germany); myocardial damage as indicated by high-sensitive cardiac troponin I (hs-cTnI, Abbott laboratories, Abbott Park, IL, USA) and copeptin (Thermo-Fisher B.R.A.H.M.S. AG, Hennigsdorf, Germany); cardiac load as indicated by BNP (Abbott laboratories, Abbott Park, IL, USA); and mid regional pro-atrial natriuretic peptide (MR-proANP, Thermo-Fisher B.R.A.H.M.S. AG, Hennigsdorf, Germany). After discharge from the ED/hospital, all patients were followed-up at 30 days (30-day) via phone interview by a trained medical student or a clinical research associate. The primary end point was 30-day all-cause mortality.

Mean plasma concentrations of cardiovascular biomarkers were elevated in AECOPD patients at admission, as shown in Table 3.

**Table 3**

| | **Available** | **Alive** | **Dead** | **p value** | **Normal Value** |
|---|---|---|---|---|---|
| Hs-cTnI, ng/L | 335 (94) | 16 (7.6 to 44) | 40.3 (12.9 to 168) | 0.0088 | < 16 (F), < 34 (M) |
| Copeptin, pmol/L | 309 (87) | 13.3 (6.1 to 33) | 30.9 (16 to 62.9) | 0.002 | < 28.2 |
| BNP, pg/mL | 336 (94) | 77 (23 to 230) | 183 (79 to 584) | 0.007 | < 100 |
| MR-proANP, pmol/L | 163 (46) | 145 (64 to 264) | 297 (147 to 549) | 0.007 | < 85.2 |
| PCT, µg/L | 309 (87) | 0.1 (0.1 to 0.2) | 0.2 (0.1 to 0.5) | 0.028 | < 0.5 |
| CRP, mg/L | 280 (79) | 22 (6 to 64) | 36 (9 to 99) | 0.21 | < 5 |

Elevated plasma hs-cTnI was found in 130 patients (36.5%), elevated copeptin was found in 98 patients (27.5%), and the combination of elevated hs-cTnI and copeptin was found in 49 patients (13.8%). BNP was elevated in 146 patients (41%), MR-proANP was elevated in 107 patients (30.1%), PCT was elevated in 32 patients (9%), and CRP was elevated in 250 patients (70.2%).

Furthermore, hs-cTnI, copeptin, NPs (both BNP and MR-proANP) and PCT levels were significantly greater at admission in 30-day non-survivors than in survivors (see Table 3). However, CRP plasma concentrations were similar at admission between non-survivors and survivors.

When assessing association with 30-day mortality by ROC analyses, all measured biomarkers displayed similar AUCs, sensitivity, specificity, negative predictive value (NPV), and positive predictive value (PPV) as observed for the CURB-65 score.

Multiple logistic regression indicated that three cardiovascular biomarkers - hs-cTnI, copeptin, and BNP - were independently associated with 30-day mortality, whether analyzed as continuous variables or dichotomized according to the threshold found by ROC curve analysis, as shown in Table 4.

**Table 4**

| | | OR [95% CI] | p value |
|---|---|---|---|
| Hs-cTnI, ng/L | *continuous* | 1.004 [1.001 - 1.006] | 0.007 |
| Copeptin, pmol/L | *continuous* | 1.005 [1.001 - 1.009] | 0.023 |
| BNP, pg/mL | *continuous* | 1.001 [1.000 - 1.001] | 0.004 |
| MR-proANP, pmol/L | *continuous* | 1.001 [1.000 - 1.002] | 0.066 |
| PCT, µg/L | *continuous* | 0.956 [0.819 - 1.117] | 0.571 |
| CRP, mg/L | *continuous* | 1.003 [0.998 - 1.008] | 0.230 |

hs-cTnI alone offered incremental prognostic value when associated with the CURB-65 score, with a total NRI of 61.6% (21.3% to 41.3%) better than incremental values of copeptin or BNP alone, as shown in Table 5.

**Table 5**

| | **HS-cTnI** | **Copeptin** | **HS-cTnl+Copeptin** | **BMP** |
|---|---|---|---|---|
| ***NRI*** | | | | |
| % Events to higher risk | 39.1% | 30.4% | 43.5% | 39.1% |
| % Events to lower risk | 8.3% | 11.5% | 10.8% | 10.5% |
| % Nonevents to higher risk | 60.9% | 69.6% | 56.5% | 60.9% |
| % Nonevents to lower risk | 91.7% | 88.5% | 89.2% | 89.5% |
| Total NRI for events [95% CI] | -0.217 [-0.349 to 0.4481 | -0.391 [-0.323 to 0.4291 | -0.13 [-0.331 to 0.481 | -0.217 [-0.344 to 0.454] |
| Total NRI for nonevents [90% CI] | 0.834 [-0.012 to 0.111] | 0.769 [-0.021 to 0.127] | 0.783 [0.003 to 0.147] | 0.789 [-0.013 to 0.123] |
| Total NRI [95% CI] | 0.616 [0.213 to 0.453] | 0.378 [-0.005 to 0.436] | 0.653 [0.241 to 0.486] | 0.572 [0.167 to 0.46] |
| ***IDI*** | | | | |
| Events to higher risk | 0.040 | 0.048 | 0.068 | 0.045 |
| Nonevents to lower risk | 0.010 | 0.005 | 0.007 | 0.01 |
| Total [95% CI] | 0.049 [-0.008 to 0.107] | 0.053 [-0.013 to 0.119] | 0.075 [-0.002 to 0.151] | 0.055 [-0.001 to 0.112] |

More important, the combination of hs-cTnI and copeptin exhibited an improved incremental prognostic value when associated with CURB-65 scores as compared to hs-cTnI alone 65.3% (24.1 % to 48.6%). IDI was also higher for the combination of hs-TnI and copeptin as compared to hs-TnI, copeptin, or BNP alone. The combination of elevated hs-cTnI and elevated copeptin (n=49, 16%) also was associated with a very high 30-day mortality (> 20%) (see Figure 1), which was more than 3-fold greater than elevated hs-cTnI and normal copeptin levels (5.7%), normal hs-cTnI and high copeptin levels (6.1%), or normal hs-cTnI and normal copeptin levels (4.3%).

This study confirmed the expected association between advanced lung dysfunction and short-term mortality. Low arterial oxygen saturations and high CURB-65 scores were associated with a high risk of short-term mortality. This study also confirmed that hs-cTnI, but not natriuretic peptides or PCT-CRP, added prognostic value to the CURB-65 pulmonary severity score for prediction of 30-day mortality in AECOPD. AECOPD cases with elevated plasma concentrations of both troponin and copeptin had the poorest 30-day outcome.

Accordingly, the results of this example demonstrate that the association of hs-troponin and copeptin identifies AECOPD patients with a poor prognosis, which potentially reflects myocardial damage that should trigger additional management efforts to rule out or treat AMI or critical cardiac ischemia.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method of detecting cardiac troponin (cTn) and copeptin proteins in a sample obtained from a patient diagnosed with chronic obstructive pulmonary disease (COPD), which method comprises:
(a) obtaining a blood sample from a patient diagnosed with COPD,
(b) detecting whether cardiac troponin and copeptin are present in the blood sample by contacting the blood sample with an anti-cardiac troponin antibody and an anti-copeptin antibody.

2. The method of claim 1, wherein the patient is diagnosed with an acute exacerbation of COPD (AECOPD).

3. A method of determining risk of short-term mortality in a patient having an acute exacerbation of COPD (AECOPD), which method comprises:
(a) obtaining a blood sample from a patient diagnosed with AECOPD,
(b) contacting the blood sample with an anti-cardiac troponin antibody and an anti-copeptin antibody,
(c) detecting binding of cardiac troponin to the anti-cardiac troponin antibody and binding of copeptin to the anti-copeptin antibody,
(d) quantifying the amounts of cardiac troponin and copeptin in the blood sample, wherein increased levels of cardiac troponin and copeptin in the blood sample as compared to normal levels of cardiac troponin and copeptin indicate an increased risk of short-term mortality in the patient.

4. The method of claim 3, which further comprises determining the CURB-65 score of the patient.

5. The method of claim 4, wherein the patient comprises a CURB-65 score of between 3 and 5.

6. The method of any one of claims 3-5, wherein the patient is hospitalized for AECOPD and short-term mortality is death of the patient within 30 days of hospitalization.

7. The method of any one of claims 3-6, wherein a patient having an increased risk of short-term mortality is diagnosed with acute myocardial injury (AMI).

8. The method of claim 7, further comprising treating the AMI in the patient.

9. The method of any one of claims 3-8, wherein an increased risk of short-term mortality in the patient is indicated when the level of cardiac troponin in the patient is at least 2-fold greater than the normal level of cardiac troponin and the level of copeptin in the patient is at least 2-fold greater than the normal level of copeptin.

10. The method of any one of claims 1-9, wherein the blood sample is plasma.

11. The method of any one of claims 1-10, wherein the cardiac troponin is cardiac troponin I (cTnI).

12. The method of any one of claims 1-11, wherein binding of cardiac troponin to the anti-cardiac troponin antibody is detected with a high-sensitivity cardiac troponin assay (hs-cTn).
